# FASCICULE DE BREVET EUROPEEN

(11) **EP 0 563 486 B1**
(45) Date de publication et mention de la délivrance du brevet: **30.07.1997**
(21) Numéro de dépôt: 92400884.0
(22) Date de dépôt: 30.03.1992
(51) Int. Cl.: B05C 17/01, B05B 7/24, A61M 35/00

(54) **Dispositif pour appliquer une composition pharmaceutique ou cosmétique**
Vorrichtung zum Auftragen einen pharmazeutischen oder kosmetischen Zusammensetzung
Device for applying a pharmaceutical or cosmetic composition

(43) Date de publication de la demande: 06.10.1993
(73) Titulaire: IMMUNO FRANCE S.A.R.L., F-94577 Rungis Cédex (FR)
(72) Inventeur: Kerbrat, Renaud, F-14610 Thaon Epron (FR)
(74) Mandataire: De Palmenaer, Roger

(56) Documents cités:
- EP-A- 0 148 980
- EP-A- 0 343 003
- EP-A- 0 463 990
- US-A- 3 208 638
- US-A- 4 174 068
- US-A- 4 544 369

## Description

La présente invention est relative à un dispositif pour appliquer, déposer ou répartir une composition pharmaceutique ou cosmétique en particulier sur un organisme ou une partie de celui-ci.

Des dispositifs pour déposer une composition sont connus.

Par le document US-A-3,208,638, ou connait un dispositif pour déposer une quantité d'une composition sur un support, ce dispositif comprenant un moyen destiné à modifier le volume d'un réservoir contenant ladite composition de manière à l'amener vers un organe de distribution ou répartition. Le dispositif comprend une carcasse, capable d'être tenue dans une seule main par un utilisateur, un support pour ledit réservoir et une pièce mobile par rapport à la carcasse destinée à modifier le volume du réservoir, ladite pièce mobile étant actionnée par un moteur, de préférence à vitesse variable commandé par une détente actionnée par un doigt de ladite main ou une partie de ladite main et par un dispositif de régulation de la vitesse du moteur ou d'un arbre entraîné par celui-ci, de manière à déposer une quantité déterminée de la composition sur un support.

De tels dispositifs sont connus.

On connait également un dispositif ayant la forme d'un tube. La composition pharmaceutique ou cosmétique sort de façon irrégulière du tube en pressant sur une extrémité de celui-ci et ne peut être répartie sur l'organisme que de façon irrégulière au moyen de la main ou d'une palette.

Dans une autre forme de réalisation connue, le dispositif comprend :
(a) un réservoir dont le volume peut être modifié par un piston solidaire d'une tige, ledit piston se déplaçant dans ledit réservoir, et
(b) un organe de distribution constitué d'un pulvérisateur relié à une source de gaz comprimé. Pour utiliser un tel dispositif, l'utilisateur doit à l'aide d'une main appliquer un effort sur la tige de manière à amener la composition contenue dans le réservoir vers l'organe de distribution et à l'aide d'un pied commander l'arrivée de gaz à l'organe de distribution.

Un tel dispositif présente les inconvénients suivants :
- Il n'est pas possible d'assurer un débit sensiblement constant de la composition vers l'organe de distribution ou un débit très faible de ladite composition. Le film obtenu par un tel dispositif est épais et fort irrégulier.
- Le chirurgien qui utilise un tel dispositif pour arrêter des hémorragies par application d'une colle plasmatique est en position debout. Dés lors, il ne lui est uniquement possible par un mouvement de son pied d'arrêter ou de permettre l'admission de gaz dans l'organe de distribution, c'est-à-dire qu'il lui est impossible de réguler le débit de gaz.

De plus, dû à la fatigue lors d'une opération, la maîtrise de l'appareil par le chirurgien est affectée de sorte que le débit de composition sortant du réservoir a tendance d'être trop important.

Enfin, dû à la viscosité de la composition, en particulier d'une colle plasmatique, le chirurgien a tendance à appliquer un effort trop important sur la tige de sorte que lorsque la composition sort du réservoir (la viscosité étant alors réduite dû au mouvement de la composition dans le réservoir) une quantité trop importante de composition continue à être amenée à l'organe de distribution. Dans ce cas, les risques de formation de gros bourrelets de colle sont importants. De tels bourrelets ne permettent pas d'obtenir, dans le cas de la colle plasmatique, une bonne adhésion de celle-ci. De plus, la formation de gros bourrelets, c'est à dire de quantités trop importantes de produit, peut provoquer l'apparition d'effets secondaires.

La présente invention vise à remédier à ces inconvénients. Elle a pour objet un dispositif d'utilisation aisée, par exemple, qui peut être tenu et commandé par une seule main, dispositif convenant pour appliquer, distribuer ou répartir d'une façon régulière une composition pharmaceutique ou cosmétique sur un organisme ou une partie de celui-ci.

La présente invention a également pour objet un dispositif convenant pour réaliser sur un organisme ou une partie de celui-ci un film régulier dont l'épaisseur peut être inférieure à 1 mm. De tels films adherent convenablement, par exemple, sur la peau d'un organisme et suivent correctement les mouvements de celle-ci.

Le dispositif suivant l'invention comprend un moyen destiné à modifier le volume d'un réservoir contenant une composition pharmaceutique ou cosmétique à appliquer, distribuer ou déposer sur un organisme ou partie d'organisme, ce moyen amenant ladite composition vers un organe de distribution ou de répartition. Le dispositif suivant l'invention comprend en outre une carcasse capable d'être tenue dans une seule main par un utilisateur, un support pour ledit réservoir et une pièce mobile par rapport à la carcasse destinée à modifier le volume du réservoir. Ladite pièce mobile est actionnée par un moteur, de préférence à vitesse variable, commandé
(*) par une détente actionnée par un doigt ou une partie de la main de l'utilisateur, et
(*) par un dispositif de régulation de la vitesse du moteur ou d'un arbre entraîné par celui-ci, de manière à déposer une quantité déterminée de la composition d'une manière déterminée, en particulier sur l'organisme ou une partie de celui-ci. Selon l'invention, l'organe de distribution est un pulverisateur.

Le dispositif de régulation est dans une forme de réalisation un dispositif de régulation de l'énergie fournie au moteur. Ce dispositif de régulation est avantageusement commandé par la gâchette ou détente.

Dans une forme de réalisation particulière du dispositif suivant l'invention, le moteur entraîne, avantageusement via un réducteur, une vis sans fin dont une partie est engagée dans un sabot de poussée, le dispositif présentant un système de guidage du sabot par rapport à la carcasse de manière à permettre la transformation du mouvement de rotation de la vis sans fin en un mouvement sensiblement rectiligne du sabot.

Selon une particularité d'une forme de réalisation, la gâchette commande un potentiomètre situé entre une source de courant et le moteur, l'actionnement de ladite gâchette permettant de modifier la tension appliquée au moteur et donc sa vitesse de rotation.

L'organe de distribution et de pulverisation peut comprendre un conduit tel qu'un capillaire présentant une diamètre intérieur inférieur à 2,3 mm.

Selon l'invention, le dispositif est avantageusement muni d'un système d'amenée de gaz vers l'organe de distribution et d'un moyen pour modifier la quantité ou débit de gaz amené vers l'organe de distribution. Un tel moyen est par exemple une vanne actionnée par une gâchette que présente la carcasse, cette vanne étant montée sur un conduit dont une extrémité est destinée à être raccordée à une source de gaz, en particulier à un récipient contenant un gaz sous pression (air stérilisé, azote,...).

Le dispositif suivant l'invention convient en particulier pour l'application simultanée de plusieurs produits ou composants contenus dans des réservoirs distincts. Dans une forme de réalisation, le dispositif comprend dès lors un support pour au moins deux réservoirs, une ou plusieurs pièces mobiles agissant sur lesdits réservoirs de manière à en modifier leur volume.

La présente invention a encore pour objet un procédé pour répartir ou distribuer une composition cosmétique sur un organisme ou une partie de celui-ci dans lequel on utilise un dispositif suivant l'invention, en particulier un dispositif muni d'une tête de pulvérisation.

Selon ce procédé, on régule la vitesse du moteur de manière à répartir sur l'organisme ou sur une partie de celui-ci une quantité de composition cosmétique à un débit compris entre 6 ml/minute et 12 ml/minute et on régule le débit de gaz entre 5 l/minute et 10 l/minute de manière à obtenir sur l'organisme ou une partie de celui-ci une couche de ladite composition dont l'épaisseur est inférieure à 5 mm et avantageusement comprise entre 1 et 3 mm. La couche ainsi formée est de préférence régulière et a une épaisseur sensiblement constante.

Enfin l'invention a pour objet un dispositif suivant l'invention muni d'une tête de pulvérisation ou pulvérisateur pour la préparation d'un film destiné au traitement d'un organisme ou d'une partie de celui-ci. Un tel film a de préférence une épaisseur sensiblement constante, épaisseur avantageusement comprise entre 1 et 2 mm.

Un tel film peut être utilisé pour le traitement de brûlures (pour recouvrir les brûlures et empècher le dépôt d'impuretés sur celles-ci). De tels films, vu leur faible épaisseur, ont une certaine porosité et permettent ainsi une respiration de la peau.

Un tel film peut être également utilisé pour traiter des coupures, hémorragies, etc. Dans ce cas, une composition bi- ou multi-composants est avantageusement projetée sur la coupure, hémorragie, etc. Une telle composition est, par exemple, une colle plasmatique dont un premier composant est choisi parmi les protéines coagulables tandis que le deuxième composant est de la thrombine.

Le dispositif suivant l'invention convient de façon toute particulière pour l'application de compositions à plusieurs composants, dont les composants ne doivent être mélangés que juste avant leur application pour accroître la durée de vie desdits composants (éviter des dégradations de ceux-ci par interaction des composants entre eux) et donc l'efficacité de la composition.

D'autres particularités et détails de l'invention ressortiront de la description détaillée suivante dans laquelle il est fait référence aux dessins ci-annexés.

Dans ces dessins :
- la figure 1 est une vue en coupe d'une forme de réalisation préférée d'un dispositif suivant l'invention ;
- la figure 2 est une vue de côté du dispositif montré à la figure 1 ;
- la figure 3 est une vue partiellement en coupe d'un réservoir convenant pour un dispositif suivant l'invention, et
- la figure 4 est une vue schématique du circuit électrique commandant le moteur du dispositif représenté à la figure 1.

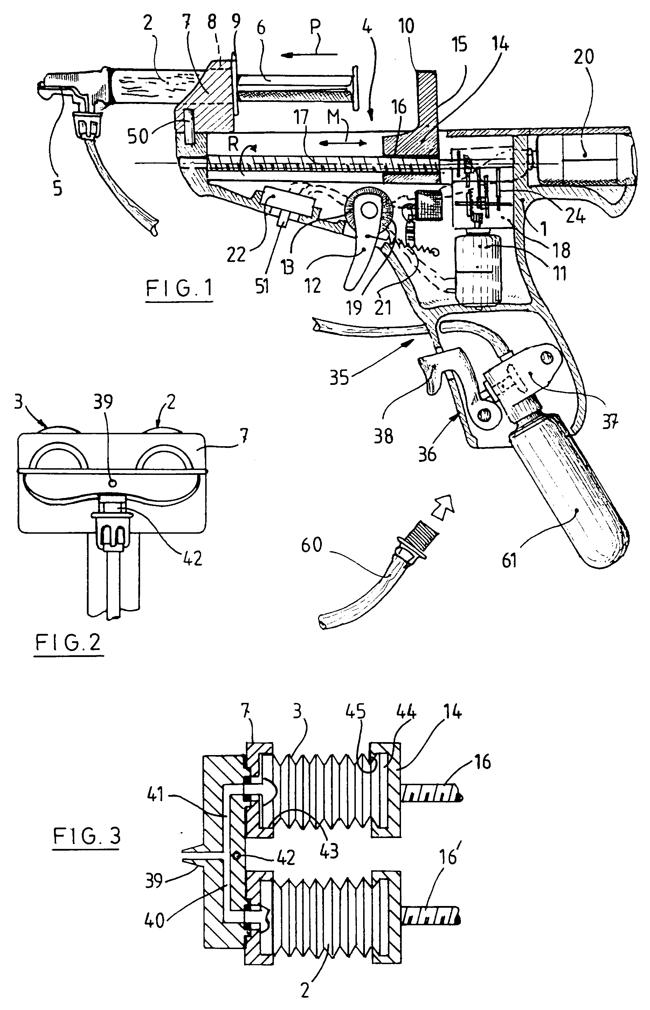

Le dispositif suivant l'invention représenté à titre d'exemple uniquement à la figure 1 comprend une carcasse 1 dont la forme générale est sensiblement celle d'un révolver ou pistolet. Une telle forme permet d'assurer une bonne préhension de l'appareil et donc facilite l'utilisation de celui-ci, en particulier lors d'opérations chirurgicales de longues durées.

Le dispositif représenté aux figures 1 et 2 est destiné à appliquer sur un organisme par exemple sur la peau de celui-ci une composition à deux composants. Un premier composant est contenu dans un réservoir 2, tandis que le deuxième composant est contenu dans le réservoir 3. Ces réservoirs 2 et 3 sont constitués par les chambres d'une seringue munie d'un piston prolongé par une tige 6.

Le dispositif comporte un moyen désigné dans son ensemble par la notation de référence 4 destiné à modifier le volume des réservoirs, c'est-à-dire dans le cas représenté, à appliquer une poussée sur la tige et le piston, de manière à amener ledit composant vers l'organe de répartition 5.

Dans le cas représenté, lesdits réservoirs 2,3 sont portés par un support 7 présentant deux canaux 8 dans lesquels sont engagés lesdits réservoirs. La forme des canaux épouse la forme extérieure des réservoirs, tandis qu'une butée 9 solidaire des réservoirs vient prendre appui sur le support 7 de manière à assurer le maintien en place des réservoirs 2,3 lorsqu'une poussée P est appliquée sur les tiges 6. Ce support est avantageusement fixé sur la carcasse 1 par des ergots 50 de sorte que celui-ci peut être solidaire des réservoirs et peut être jeté lorsque les réservoirs sont vides.

Le moyen 4 destiné à modifier le volume des réservoirs 2,3 comprend une pièce mobile 10 actionnée par un moteur 11 commandé par une détente 12 sur laquelle un doigt, de préférence l'index, de l'utilisateur agit et par un dispositif de régulation 13 de la vitesse du moteur 11 ou d'un arbre entraîné par celui-ci. Ledit moteur 11 et ledit dispositif de régulation 13 permettent de déposer une quantité déterminée de la composition contenue dans le réservoir 2 et de la composition contenue dans le réservoir 3 sur un organisme ou une partie de celui-ci.

Le moteur 11 est avantageusement un moteur à vitesse variable, tandis que le dispositif de régulation 13 est de préférence un dispositif de régulation de l'énergie apportée au moteur 11, dispositif 13 avantageusement commandé par la gächette ou détente 12.

La pièce mobile 10 est constituée d'un sabot de poussée 14 portant un bras 15 destinée à agir sur les tiges 6 des réservoirs 2,3. Le sabot 14 présente un canal fileté 16 dans lequel est engagée une tige filetée ou vis sans fin 17 entraînée par le moteur 11 via un réducteur 18.

Pour obtenir un mouvement sensiblement rectiligne du sabot, le dispositif suivant l'invention est muni d'un système de guidage du sabot par rapport à la carcasse. Un tel système est par exemple un ergot solidaire du sabot, ledit ergot étant engagé dans une rainure que présente la carcasse 1. Ce système permet la transformation du mouvement de rotation R de la vis sans fin 17 en un mouvement M sensiblement rectiligne du sabot 14.

Dans une forme de réalisation représentée aux figures 1 et 2, le dispositif de régulation comprend un potentiomètre 19 située entre une source de courant 20 et le moteur 11, ledit potentiomètre 19 étant commandé par la détente ou gâchette 12, de sorte qu'en fonction de la pression exercée par un doigt de l'utilisateur sur la détente, il est possible de modifier la tension appliquée au moteur 11 et donc sa vitesse de rotation.

Le dispositif suivant l'invention est muni d'un moyen de rappel 21 agissant sur la détente 12, ce moyen de rappel est destiné a ramener la détente 12 lorsqu'aucune pression n'est appliquée sur celle-ci dans une position dans laquelle le moteur n'est pas actionné.

Selon une particularité du dispositif représenté, il est muni d'un inverseur 22 destiné à permettre au moteur d'éloigner la pièce mobile ou sabot 14 du support 7. Cet inverseur 22 comprend un contacteur à bouton 51. Lorsqu'une pression est exercée sur ledit bouton 51, un courant est amené de la source 20 au moteur 11 sans passer par le potentiomètre. La différence de potentiel appliquée par ledit inverseur au moteur est alors négative lorsque la différence de potentiel appliquée par le potentiomètre est positive, de sorte que ledit inverseur permet de tourner en sens inverse et donc permet l'éloignement du sabot 15 par rapport au support 7.

La source de courant est constituée par une batterie ou pile, par exemple une pile de 1,5 V ou de 9 V, cette pile étant logée dans une évidement 24 que présente la carcasse 1.

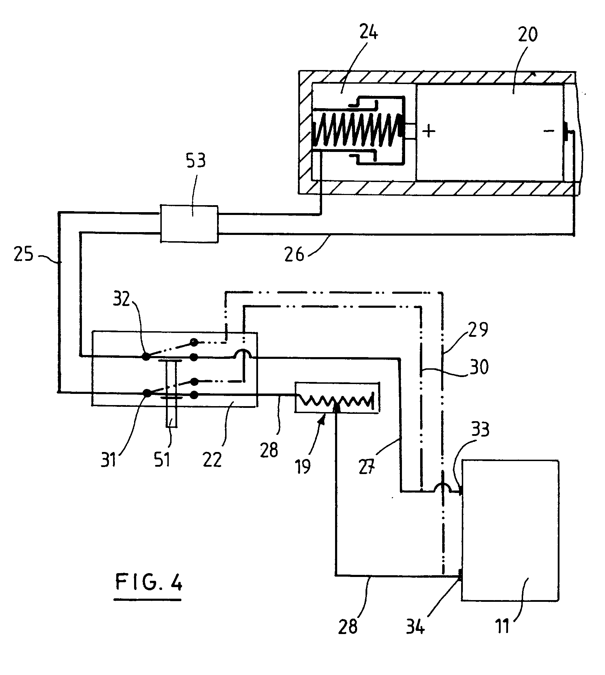

Le circuit électrique commandant le dispositif représenté est le suivant (voir figure 4).

La pile 20 logée dans l'évidement 24 est reliée à l'inverseur 22 par deux fils 25, 26 sur lesquels est montée une diode 53. L'inverseur 22 est relié au moteur 11 par quatre fils 27, 28, 29 et 30.

Un premier couple de fils 27, 28 relie l'inverseur 22 au moteur via le potentiomètre 19 de manière à permettre le déplacement du sabot 15 vers le support 7.

Un deuxième couple de fils 29, 30 permet d'amener de l'énergie électrique au moteur 11 sans passer par le potentiomètre 19.

L'inverseur 22 comprend un premier contact d'entrée 31 relié par le fil 25 au pôle positif de la pile 20 et un deuxième contact d'entrée 32 relié par le fil 26 au pôle négatif de la pile 23. Lorsqu'aucune pression n'est exercée sur le bouton 51 (position représentée à la figure 4), le pôle négatif de la pile 20 est relié à un premier plot 33 du moteur 11 par le fil 27, tandis que le pôle positif de la pile est relié par le fil 28 et le potentiomètre 19 au deuxième plot 34 du moteur 11.

Lorsqu'une pression est exercée sur le bouton 51, les contacts d'entrée 31, 32 de l'inverseur sont reliés respectivement aux fils 29 et 30. Ainsi, le pôle négatif de la pile 20 est relié par le fil 29 au plot 34 du moteur, tandis que le pôle positif de la pile est relié par le fil 30 au plot 33 du moteur, de sorte que le moteur peut tourner dans le sens inverse au sens de rotation du moteur lorsque le bouton 51 est dans la position représentée à la figure 4.

Le dispositif suivant l'invention est muni d'un dispositif de distribution ou organe de répartition 5. Ce dispositif ou organe qui peut comprendre un conduit ou un capillaire ayant un diamètre intérieur inférieur à 2,4 mm (diamètre intérieur avantageusement compris entre 2 et 2,4 mm) est un pulvérisateur. Le dispositif est muni d'un système d'amenée de gaz 35 vers l'organe de distribution 5 et d'un moyen 36 pour modifier par exemple à volonté, la quantité ou le débit de gaz amené à l'organe de distribution. Le moyen 36 est constitué par une vanne montée sur le conduit 37 reliant l'organe de distribution 5 à la source de gaz 37. Cette vanne 36 est actionnée par une gâchette 38 que présente la carcasse 1. Cette gâchette 38 est commandée par un doigt de l'utilisateur, par exemple par l'annulaire.

Le pulvérisateur 5 comprend une tête de pulvérisation 39 reliée par un premier canal 40 au premier réservoir 2, par un deuxième canal 41 au deuxième réservoir 3 et par un troisième canal 42 au conduit d'amenée de gaz (voir figure 3).

Le gaz qui peut être utilisé peut être de l'air par exemple de l'air stérile, de l'azote, etc, ce gaz pouvant provenir d'une source externe via un conduit 60 ou d'un récipient 61 contenant un gaz sous pression, récipient avantageusement muni d'un détendeur.

Des exemples de détendeurs sont donnés ci-après :
1°) Le détendeur pour faible pression (+ ou - 3 bars). Celui-ci peut avoir un corps en plastique et être particulièrement petit. Son déclenchement ne nécessite pas d'efforts particuliers en raison des faibles pressions à vaincre. Il est composé d'une entrée du gaz et d'une sortie calibrée et réglable, avec en intermédiaire un clapet ou valve obturant le conduit de sortie. Ce clapet est monté avec des dispositifs étanches, simples, comme un joint torique par exemple. La commande de ce clapet se fait par la saillie d'une tige hors du corps du détendeur, et suffisamment longue pour dépasser hors du corps de l'appareil lui-même. Sur cette tige, il est adapté un bouton, qui commande l'ouverture ou la fermeture du détendeur par l'action du doigt de l'utilisateur sur ce bouton.
2°) L'appareil peut être muni d'un détendeur avec réserve de gaz autonome, ce détendeur fonctionne avec des pressions élevées de l'ordre de 40 bars, l'aménagement de ce détendeur est sensiblement identique au détendeur à basse pression, mais il comporte des dispositifs d'étanchéité plus conséquents, son fonctionnement ne peut s'effectuer qu'avec un dispositif démultiplicateur compte tenu des forces à contraindre. Le corps de ce détendeur est obligatoirement en métal, ou dans certains plastiques très résistants. De toutes façons et selon l'invention, l'interchangeabilité des détendeurs est possible sur cet appareil.
3°) Le détendeur est positionné hors de l'appareil, et dans ce cas seul une valve classique remplace le détendeur miniature dans l'appareil caractérisé selon l'invention.

La figure 3 montre partiellement et en coupe une autre forme de réalisation d'un dispositif suivant l'invention.

Dans cette forme de réalisation, les réservoirs 2, 3 destinés à contenir les différents composants de la composition à pulvériser sont des réservoirs à soufflets de sorte qu'en appliquant une pression sur une extrémité du réservoir par rapport à l'autre extrémité du réservoir, les soufflets se rapprochents les uns des autres, c'est-à-dire que le volume du réservoir est modifié.

Dans cette forme de réalisation l'extrémité d'un réservoir à soufflets est placée dans une cavité 43 que présente le support 7 tandis que l'autre extrémité est placée dans une cuvette 44 formée dans le sabot 14. Le sabot 14 de chaque réservoir est actionné par une vis sans fin indépendante 16, 16' avantageusement commandée par des moteurs indépendants. Des doigts différents d'une main d'un utilisateur peuvent agir sur des détentes différentes de manière à obtenir des vitesses de rotation différentes pour chacun desdits moteurs.

La cuvette 44 du sabot est munie de tenons 45 destinés à bloquer une extrémité du réservoir, ainsi qu'à maintenir des soufflets en position rapprochée lorsqu'une pression a été exercée par le sabot 7 sur l'extrémité du réservoir.

La présente invention a encore pour objet un procédé pour répartir ou distribuer sur un organisme ou une partie de celui-ci une composition en particulier une composition cosmétique.

Selon un procédé suivant l'invention, dans lequel on utilise le dispositif représenté à la figure 1, on régule la vitesse du moteur de manière à répartir sur l'organisme ou une partie de celui-ci une quantité de composition, de préférence cosmétique, à un débit compris entre 6 ml/minute et 12 ml /minute et on régule le débit de gaz entre 5 l/minute et 10 l/minute.

De cette manière, il est possible de répartir de façon régulière une composition par exemple multi-composants en une couche ayant une épaisseur inférieure à 5 mm, de préférence comprise entre 1 mm et 3 mm.

La présente invention a encore pour objet l'utilisation d'un dispositif suivant l'invention, en particulier celui-représenté à la figure 1, pour la préparation d'un film pour le traitement d'un organisme ou une partie de celui-ci. L'épaisseur du film avantageusement déposé sur un support est dans une forme de réalisation préférée sensiblement constante, cette épaisseur est par exemple comprise entre 1 mm et 3 mm. Un tel support qui peut être muni de la composition juste avant l'emploi est par exemple une compresse de collagène résorbable.

Le dispositif suivant l'invention est particulièrement utile pour appliquer sur un organisme ou une partie de celui-ci une composition pharmaceutique sous forme de film. La composition pharmaceutique est par exemple une colle plasmatique multi-composants, par exemple bi-composants. Une telle colle peut être utilisée pour le traitement d'hémorragie, de brûlures, etc.

Le dispositif suivant l'invention en particulier lorsqu'il est muni d'une pile logée dans un évidement de la carcasse et lorsqu'il est muni d'un récipient de gaz sous pression convient au conditionnement de compositions, en particulier pharmaceutiques, multi-composants. Ce dispositif comprend des réservoirs distincts pour les différents composants de sorte que la durée de vie ou l'efficacité de la composition est accrue.

Il va de soi que de nombreuses modifications du dispositif suivant l'invention sont possibles. Ainsi, dans une forme de réalisation le débit de gaz nécessaire à la pulvérisation, ou plus exactement une détente partielle de celui-ci, peut être utilisé pour fournir l'énergie nécessaire au déplacement du sabot. L'avantage d'une telle solution est que le débit de composition pulvérisée sera fonction du débit de gaz.

Dans une autre forme de réalisation, les gachettes ou détentes sont remplacées par des capteurs de pression.

Dans encore une autre forme de réalisation, le moyen 36 pour modifier la quantité ou débit de gaz amené vers l'organe de distribution est une vanne électro-commandée. Cette vanne est par exemple commandée en fonction de la tension appliquée au moteur.

## Revendications

1. Dispositif pour déposer une quantité d'une composition pharmaceutique ou cosmétique sur un organisme ou une partie de celui-ci, ou sur un support destiné à être mis en contact avec celui-ci, ce dispositif comprenant un moyen destiné à modifier le volume d'un réservoir contenant ladite composition de manière à l'amener vers un organe de distribution ou répartition, le dispositif comprenant une carcasse (1), capable d'être tenue dans une seule main par un utilisateur, un support (7) pour ledit réservoir (2, 3) et une pièce mobile (10) par rapport à la carcasse (1) destinée à modifier le volume du réservoir (2, 3), ladite pièce mobile (10) étant actionnée par un moteur (11), de préférence à vitesse variable commandé par une détente (12) actionnée par un doigt de ladite main ou une partie de ladite main et par un dispositif de régulation (13) de la vitesse du moteur (11) ou d'un arbre entraîné par celui-ci, de manière à déposer une quantité déterminée de la composition d'une manière déterminée sur un support, sur l'organisme ou sur une partie de celui-ci, caracterisé en ce que l'organe de distribution est un pulverisateur (5)

2. Dispositif suivant la revendication 1, caractérisé en ce qu'il est muni d'un système d'amenée de gaz (35) vers l'organe de distribution (5) et d'un moyen (36) pour modifier la quantité ou débit de gaz amené vers l'organe de distribution.

3. Dispositif suivant la revendication 2, caractérisé en ce que ledit moyen (36) comporte une vanne actionnée par une gâchette (38) que présente la carcasse (1).

4. Dispositif suivant la revendication 3, caractérisé en ce que la vanne est montée sur un conduit (37) dont une extrémité est destinée à être raccordée à une source de gaz, en particulier à un récipient (61) contenant un gaz sous pression.

5. Dispositif suivant l'une quelconque des revendications 1 à 4, caractérisé en ce qu'il comprend un support (7) pour au moins deux réservoirs (2,3), une ou plusieurs pièces mobiles (10) agissant sur lesdits réservoirs (2,3) de manière à en modifier leur volume.

6. Dispositif suivant l'une des revendication 1 à 5, caractérisé en ce que le dispositif de régulation (13) est un dispositif de régulation de l'énergie apportée au moteur (11), ce dispositif étant commandé par ladite gâchette ou détente (12).

7. Dispositif suivant l'une des revendication 1 à 6, caractérisé en ce que le moteur (11) entraîne, avantageusement via un réducteur (18) une vis sans fin (17), dont une partie est engagée dans un sabot de poussée (14) et en ce que le dispositif présente un système de guidage du sabot (14) par rapport à la carcasse (1), ce système permettant la transformation du mouvement de rotation de la vis en un mouvement sensiblement rectiligne du sabot.

8. Dispositif suivant l'une quelconque des revendications 1 à 7, caractérisé en ce que la gâchette (12) commande un potentiomètre (19) situé entre une source de courant (20) et le moteur (11), l'actionnement de ladite gâchette (12) permettant de modifier la tension appliquée audit moteur (11) et donc sa vitesse de rotation.

9. Dispositif suivant l'une quelconque des revendications 1 à 8, caractérisé en ce que le sabot présente un bras (15) destiné à venir en contact avec une surface du réservoir (2,3) ou avec un élément dudit réservoir pour en modifier son volume.

10. Dispositif suivant l'une quelconque des revendications 1 à 9, caractérisé en ce que le support (7) de réservoir présente un canal (8) dont la forme épouse sensiblement la forme extérieure d'une partie du réservoir (2, 3) et une butée pour empécher un mouvement dudit réservoir (2, 3) par rapport a la carcasse lorsque le sabot (14) agit sur le réservoir (2,3) ou une partie de celui-ci.

11. Dispositif suivant l'une quelconque des revendications 1 à 10, caractérisé en ce qu'un moyen de rappel (21) agit sur la gâchette (12) de manière à ramener celle-ci, lorsqu'aucune pression n'y est appliquée, dans une position dans laquelle le moteur (11) n'est pas actionné.

12. Dispositif suivant l'une quelconque des revendications 1 à 11, caractérisé en ce qu'il est muni d'un inverseur (22) destiné à permettre au moteur (11) d'éloigner la pièce mobile (10) par rapport au support (7).

13. Dispositif suivant l'une quelconque des revendications 1 à 12, caractérisé en ce que le moteur (11) est alimenté en courant électrique provenant d'une batterie (20) logée dans un évidement (24) de la carcasse (1).

14. Dispositif suivant l'une quelconque des revendications 1 à 13, caractérisé en ce que l'organe de distribution (5) comprend un conduit, de préférence un capillaire de diamètre intérieur inférieur à 2, 3 mm.

15. Procédé pour répartir ou distribuer une composition cosmétique sur un organisme ou une partie de celui-ci dans lequel on utilise un dispositif suivant l'une quelconque des revendications précédentes, caractérisé en ce qu'on régule la vitesse du moteur de manière à répartir sur l'organisme ou une partie de celui-ci une quantité de composition cosmétique à un débit compris entre 6 ml/minute et 12 ml/minute et on régule le débit de gaz entre 5 l/minute et 10 l/minute de manière à obtenir sur l'organisme ou partie de celui-ci une couche de ladite composition dont l'épaisseur est comprise entre 1 et 3 mm.

16. Procédé suivant la revendication 15, caractérisé en ce qu'on régule la vitesse du moteur et le débit de gaz de manière à obtenir une couche d'épaisseur sensiblement uniforme.

17. Utilisation d'un dispositif suivant l'une quelconque des revendications 1 à 14, pour la préparation d'un film destiné au traitement d'un organisme ou d'une partie de celui-ci, l'épaisseur dudit film étant sensiblement constante et étant avantageusement comprise entre 1 et 3 mm.

18. Dispositif suivant l'une quelconque des revendications 1 à 14 pour appliquer sur un organisme ou une partie de celui-ci, sous forme de film une composition pharmaceutique, en particulier une colle plasmatique à deux composants.

19. Dispositif de conditionnement d'une composition en particulier pharmaceutique à plusieurs composants, ledit dispositif étant un dispositif suivant la revendication 15 et comprenant au moins deux réservoirs distincts pour contenir au moins deux composants de ladite composition, ledit dispositif permettant l'apport desdits composants vers l'organe de distribution dans un rapport déterminé.

## Claims

1. Device for depositing a quantity of a pharmaceutical or cosmetic composition on an organism or a part of it, or on a support intended to be put in contact with it, this device comprising a means intended for altering the volume of a reservoir containing the aforementioned composition in order to take it to a distribution or spreading mechanism, the device comprising a casing (1), capable of being held in one hand by a user, a support (7) for the aforementioned reservoir (2, 3) and a part (10) mobile in relation to the casing (1) intended for altering the volume of the reservoir (2, 3), the aforementioned mobile part (10) being operated by a motor (11), preferably of variable speed controlled by a trigger (12) operated by a finger of the aforementioned hand or a part of the aforementioned hand and by a speed regulating device (13) of the motor (11) or of a shaft driven by this, in order to deposit a specific quantity of the composition in a specific manner on a support, on the organism or on a part of it, characterised in that the distribution mechanism is a spray (5).

2. Device according to claim 1, characterised in that it is provided with a system for supplying gas (35) to the distribution mechanism (5) and a means (36) for altering the quantity or flow of gas supplied to the distribution mechanism.

3. Device according to claim 2, characterised in that the aforementioned means (36) comprises a valve operated by a spring catch (38) provided in the casing (1).

4. Device according to claim 3, characterised in that the valve is mounted on a duct (37) of which one extremity is intended to be connected to a gas source, in particular to a recipient (61) containing a gas under pressure.

5. Device according to any one of the claims 1 to 4, characterised in that it comprises a support (7) for at least two reservoirs (2, 3), one or several mobile parts (10) acting on the aforementioned reservoirs (2, 3) in order to alter their volume.

6. Device according to one of the claims 1 to 5, characterised in that the regulating device (13) is a regulating device for the power supplied to the motor (11), this device being operated by the aforementioned spring catch or trigger (12).

7. Device according to one of the claims 1 to 6, characterised in that via a reduction gear (18) the motor (11) advantageously drives an endless screw (17), of which a part is engaged in a thrust block (14) and in that the device shows a block (14) guiding system in relation to the casing (1), this system enabling the transformation of the rotating movement of the screw into a considerably rectilinear movement of the block.

8. Device according to any one of the claims 1 to 7, characterised in that the spring catch (12) operates a potentiometer (19) located between a current supply (20) and the motor (11), the actuation of the aforementioned spring catch (12) enabling the alteration of the voltage applied to the aforementioned motor (11) and therefore its rotation speed.

9. Device according to any one of the claims 1 to 8, characterised in that the block shows an arm (15) intended to come into contact with a surface of the reservoir (2, 3) or with an element of the aforementioned reservoir in order to alter its volume.

10. Device according to any one of the claims 1 to 9, characterised in that the reservoir support (7) shows a channel (8) whose form considerably corresponds to the exterior form of a part of the reservoir (2, 3) and a stop for preventing a movement of the aforementioned reservoir (2, 3) in relation to the casing when the block (14) acts on the reservoir (2, 3) or a part of this.

11. Device according to any one of the claims 1 to 10, characterised in that a return means (21) acts of the spring catch (12) in order to bring this back, when no pressure is applied to it, into a position in which the motor (11) is not operated.

12. Device according to any one of the claims 1 to 11, characterised in that it is provided with a reverser (22) intended to enable the motor (11) to drive away the mobile part (10) in relation to the support (7).

13. Device according to any one of the claims 1 to 12, characterised in that the motor (11) is powered by electric current coming from a battery (20) housed in a hollow (24) of the casing (1).

14. Device according to any one of the claims 1 to 13, characterised in that the distribution mechanism (5) comprises a pipe, preferably a capillary one of internal diameter less than 2, 3 mm.

15. Method for spreading or distributing a cosmetic composition on an organism or a part of it in which a device according to any one of the preceding claims is used, characterised in that the speed of the motor is regulated in order to spread on the organism or a part of it a quantity of cosmetic composition at a flow of between 6 ml/minute and 12 ml/minute and the flow of gas is regulated between 5 l/minute and 10 l/minute in order to obtain on the organism or part of it a layer of the aforementioned composition whose thickness is between 1 and 3 mm.

16. Method according to claim 15, characterised in that the speed of the motor and the flow of the gas are regulated in order to obtain a layer of considerably uniform thickness.

17. Utilisation of a device according to any one of the claims 1 to 14, for the preparation of a film intended for the treatment of an organism or of a part of it, the thickness of the aforementioned film being considerably constant and being advantageously comprised between 1 and 3 mm.

18. Device according to any one of the claims 1 to 14 for applying on an organism or a part of it, a pharmaceutical composition in film form, in particular a two-component plasmatic adhesive.

19. Packaging device for a composition in particular a pharmaceutical one with several components, the aforementioned device being a device according to claim 18 and comprising at least two distinct reservoirs for containing at least two components of the aforementioned composition, the aforementioned device enabling the supply of the aforementioned components to a distribution mechanism in a specific ratio.

## Patentansprüche

1. Vorrichtung zum Aufbringen einer Menge einer pharmazeutischen oder kosmetischen Zusammensetzung auf einem Organismus oder einem Teil desselben, oder auf einem Träger, der mit demselben in Kontakt zu bringen ist, welche Vorrichtung ein Mittel zum Modifizieren des Volumens eines die Zusammensetzung enthaltenden Behälters aufweist, um sie zu einem Verteil- oder Aufteilorgan zu führen, wobei die Vorrichtung ein Gehäuse (1), das von einer Bedienungsperson in einer Hand gehalten werden kann, einen Träger (7) für den Behälter (2, 3) und einen in bezug auf das Gehäuse (1) beweglichen Teil (10) aufweist, der zum Modifizieren des Volumens des Behälters (2, 3) bestimmt ist, wobei der bewegliche Teil (10) von einem Motor (11) vorzugsweise mit variabler Geschwindigkeit betätigt wird, welcher von einem Abzug (12) gesteuert wird, der von einem Finger der Hand oder einem Teil der Hand und von einer Vorrichtung (13) zum Einstellen der Geschwindigkeit des Motors (11) oder einer von diesem angetriebenen Welle betätigt wird, um eine bestimmte Menge der Zusammensetzung auf bestimmte Weise auf einem Träger, auf dem Organismus oder auf einem Teil desselben aufzubringen, dadurch gekennzeichnet, daß das Verteilorgan ein Zerstäuber (5) ist.

2. Vorrichtung nach Anspruch 1, dadurch gekennzeichnet, daß sie mit einem System zum Zuführen von Gas (35) zum Verteilorgan (5) und einem Mittel (36) zum Modifizieren der Durchflußmenge des zum Verteilorgan geführten Gases versehen ist.

3. Vorrichtung nach Anspruch 2, dadurch gekennzeichnet, daß das Mittel (36) ein Ventil aufweist, das von einem Drücker (38) betätigt wird, den das Gehäuse (1) aufweist.

4. Vorrichtung nach Anspruch 3, dadurch gekennzeichnet, daß das Ventil an einer Leitung (37) angebracht ist, von der ein Ende dazu bestimmt ist, mit einer Gasquelle, insbesondere einem Gefäß (61), das ein Gas unter Druck enthält, verbunden zu werden.

5. Vorrichtung nach einem der Ansprüche 1 bis 4, dadurch gekennzeichnet, daß sie einen Träger (7) für zumindest zwei Behälter (2, 3) aufweist, wobei ein oder mehrere bewegbare Teile (10) auf die Behälter (2, 3) einwirken, um ihre Volumen zu modifizieren.

6. Vorrichtung nach einem der Ansprüche 1 bis 5, dadurch gekennzeichnet, daß die Einstellvorrichtung (13) eine Vorrichtung zum Einstellen der Energie ist, die dem Motor (11) zugeführt wird, wobei diese Vorrichtung vom Drücker oder Abzug (12) gesteuert wird.

7. Vorrichtung nach einem der Ansprüche 1 bis 6, dadurch gekennzeichnet, daß der Motor (11), vorteilhafterweise über ein Untersetzungsgetriebe (18), eine Schnecke (17) antreibt, von der ein Teil in einen Schlitten (14) eingreift, und daß die Vorrichtung ein Führungssystem für den Schlitten (14) in bezug auf das Gehäuse (1) aufweist, wobei dieses System die Umwandlung der Drehbewegung der Schnecke in eine im wesentlichen geradwinkelige Bewegung des Schlittens ermöglicht.

8. Vorrichtung nach einem der Ansprüche 1 bis 7, dadurch gekennzeichnet, daß der Drücker (12) ein Potentiometer (19) steuert, das zwischen einer Stromquelle (20) und dem Motor (11) angeordnet ist, wobei die Betätigung des Drückers (12) das Modifizieren der an den Motor (11) angelegten Spannung und daher seiner Drehzahl ermöglicht.

9. Vorrichtung nach einem der Ansprüche 1 bis 8, dadurch gekennzeichnet, daß der Schlitten einen Arm (15) aufweist, der dazu bestimmt ist, mit einer Oberfläche des Behälters (2, 3) oder mit einem Element des Behälters in Kontakt zu kommen, um sein Volumen zu modifizieren.

10. Vorrichtung nach einem der Ansprüche 1 bis 9, dadurch gekennzeichnet, daß der Träger (7) des Behälters einen Kanal (8) aufweist, dessen Form im wesentlichen zur äußeren Form eines Teils des Behälters (2, 3) und einem Anschlag zur Verhinderung einer Bewegung des Behälters (2, 3) in bezug auf das Gehäuse paßt, wenn der Schlitten (14) auf den Behälter (2, 3) oder einen Teil desselben einwirkt.

11. Vorrichtung nach einem der Ansprüche 1 bis 10, dadurch gekennzeichnet, daß ein Rückholmittel (21) auf den Drücker (12) wirkt, um denselben, wenn kein Druck ausgeübt wird, in eine Position zurückzuführen, in der der Motor (11) nicht betätigt wird.

12. Vorrichtung nach einem der Ansprüche 1 bis 11, dadurch gekennzeichnet, daß sie mit einem Umschalter (22) versehen ist, der dazu bestimmt ist, dem Motor (11) ein Wegbewegen des beweglichen Teils (10) in bezug auf den Träger (7) zu ermöglichen.

13. Vorrichtung nach einem der Ansprüche 1 bis 12, dadurch gekennzeichnet, daß der Motor (11) mit elektrischem Strom gespeist wird, der von einer in einem Aufnahmeraum (24) des Gehäuses (1) angeordneten Batterie (20) stammt.

14. Vorrichtung nach einem der Ansprüche 1 bis 13, dadurch gekennzeichnet, daß das Verteilorgan (5) eine Leitung, vorzugsweise eine Kapillare mit einem Innendurchmesser von weniger als 2,3 mm, aufweist.

15. Verfahren zum Aufteilen oder Verteilen einer kosmetischen Zusammensetzung auf einem Organismus oder einem Teil desselben, bei welchem eine Vorrichtung nach einem der vorhergehenden Ansprüche verwendet wird, dadurch gekennzeichnet, daß die Geschwindigkeit des Motors eingestellt wird, um auf dem Organismus oder einem Teil desselben eine Menge einer kosmetischen Zusammensetzung mit einem Durchsatz zwischen 6 ml/Minute und 12 ml/Minute aufzubringen, und der Gasdurchsatz zwischen 5 l/Minute und 10 l/Minute eingestellt wird, um auf dem Organismus oder dem Teil desselben eine Schicht der Zusammensetzung zu erhalten, deren Dicke zwischen 1 und 3 mm beträgt.

16. Verfahren nach Anspruch 15, dadurch gekennzeichnet, daß die Geschwindigkeit des Motors und der Gasdurchsatz eingestellt werden, um eine Schicht mit im wesentlichen gleichmäßiger Dicke zu erhalten.

17. Verwendung einer Vorrichtung nach einem der Ansprüche 1 bis 14 für die Herstellung eines Films, der zur Behandlung eines Organismus oder eines Teils desselben bestimmt ist, wobei die Dicke des Films im wesentlichen konstant ist und vorteilhaft zwischen 1 und 3 mm beträgt.

18. Vorrichtung nach einem der Ansprüche 1 bis 14 zum Aufbringen einer pharmazeutischen Zusammensetzung, vorzugsweise eines Zweikomponenten-Plasmaklebers, in Form eines Films auf einem Organismus oder einem Teil desselben.

19. Vorrichtung zum Konditionieren einer, insbesondere pharmazeutischen, Mehrkomponenten-Zusammensetzung, welche Vorrichtung eine Vorrichtung nach Anspruch 18 ist und zumindest zwei einzelne Behälter aufweist, um zumindest zwei Komponenten der Zusammensetzung zu enthalten, welche Vorrichtung das Zuführen dieser Komponenten zum Verteilorgan in einem bestimmten Verhältnis ermöglicht.
